# EUROPEAN PATENT SPECIFICATION

(11) **EP 2 120 908 B1**
(45) Date of publication and mention of the grant of the patent: **25.01.2017**
(21) Application number: 07797371.7
(22) Date of filing: 08.05.2007
(51) Int. Cl.: A61K 31/145, A61K 31/191, A61K 33/24, A61K 9/00, A61K 47/34, A61K 47/36, A61K 47/38, A61K 47/42, A61K 47/44, A61K 31/205, A61P 27/16, A61P 31/04

(54) **SOLVATING SYSTEM AND SEALANT FOR MEDICAL USE**
SOLVATISATIONSSYSTEM UND DICHTUNGSMITTEL ZUR MEDIZINISCHEN VERWENDUNG
SYSTÈME DE SOLVATATION ET AGENT D'ÉTANCHÉITÉ POUR UNE UTILISATION MÉDICALE

(30) Priority: 08.02.2007 US 704115; 24.04.2007 US 739480; 24.04.2007 US 739528
(43) Date of publication of application: 25.11.2009
(60) Divisional application: 13194128.8
(73) Proprietor: Medtronic Xomed, Inc., Jacksonville, FL 32216-0980 (US)
(72) Inventor: HISSONG, James, B., Jacksonville, FL 32224 (US); OLIVER, Dana, A., Jacksonville, FL 32256 (US); LEWIS, Cecil, O., Jacksonville, FL 32250 (US); MYNTTI, Matthew, F., St. Augustine, FL 32092 (US); TIJSMA, Edze, Jan, NL-6224 LD Maastricht (NL); GONZALES LOPEZ, Maria Nieves, NL-6211 SW Maastricht (NL); SAUNIER, Janis, Ponte Vedra Beach, FL 32082 (US)
(74) Representative: Golding, Louise Ann
(86) International application number: PCT/US2007/068470
(87) International publication number: WO 2008/097317

(56) References cited:
- EP-A1- 0 933 081
- WO-A-98/09622
- WO-A-2006/026083
- WO-A-2006/029255
- WO-A-2006/099386
- US-A1- 2004 214 753
- US-A1- 2006 035 808

## Description

### FIELD OF THE INVENTION

This invention relates to a medicament system for use in the treatment of bacterial ear, nose or throat conditions including chronic otitis media with effusion (COME), Recurrent Acute Otitis Media (RAOM), cholesteatoma, chronic rhinosinusitis (CRS), pediatric chronic sinusitis, tonsillitis, adenoiditis, sleep apnea and chronic strep throat.

### BACKGROUND

COME and RAOM are inflammatory diseases of the middle ear. Biofilm formation may be a factor in the pathogenesis of COME, see Post, J.C., "Direct evidence of bacterial biofilms in otitis media", Laryngoscope 111(12):2083-94 (2001), Ehrlich et al., "Mucosal Biofilm Formation on Middle-Ear Mucosa in the Chinchilla Model of Otitis Media", JAMA 287(13):1710-15 (2002) and Fergie, N et al., "Is otitis media with effusion a biofilm infection?", Clin Otolaryngol Allied Sci. 29(1):38-46 (2004). Biofilms form when bacteria interact with a surface to form polymeric films (sometimes referred to as exopolysaccharide or extracellular polysaccharide polymers) that coat the surface and provide a living colony for further bacterial proliferation. Bacteria lodged in biofilms are much more difficult to remove or kill than bacteria in a plaktonic (suspended) state, and are extremely resistant to many antibiotics and biocides. Both the extracellular polysaccharide (EPS) matrix and the toxins produced by a number of different bacteria have been shown to cause inflammation by the host. It appears that the chronic inflammation associated with COME and RAOM is a host response to the bacterial biofilm.

COME and RAOM are usually initially treated using oral antibiotics and then, if need be, are more aggressively treated by placement of a tympanostomy tube. Occasionally in cases involving severe infection or high mucous content in the middle ear, the middle ear may be irrigated (e.g., with saline solution). While tympanostomy tubes work on most patients, about 20% of patients who undergo primary tympanostomy tube placement require an additional surgery (an adenoidectomy, a second set of tympanostomy tubes, and usually both an adenoidectomy and tympanostomy tube placement) to treat persistent COME or persistent RAOM.

Cholesteatoma is another ear disease condition of concern. Although generally thought to be primarily a cyst comprised of dermal cells, bacteria biofilms have also been implicated in this disease, see Chole et al., "Evidence for Biofilm Formation in Cholesteatomas", Arch Otolaryngol Head Neck Surg. 128, pp. 1129-33 (Oct. 2002). In cholesteatoma, bacterial biofilms appear to form, incite inflammation, and cause generation of a benign tumor composed mainly of bacteria at its core and dermal cells. The tumor can erode both the ossicular chain (hearing bones) and the mastoid bone, detrimentally affecting hearing. Surgical exposure and excision is the most common treatment for cholesteatoma removal. Up to 25% of these procedures fail due to recurrence of the cholesteatoma and thus require additional surgery or other treatment.

CRS is inflammation of the paranasal sinuses and is associated with anterior or posterior nasal discharge, nasal obstruction or facial pressure, pain or fullness lasting for at least about twelve weeks. CRS affects an estimated 10% or more of the U.S. population. Most patients with CRS are initially treated with medical therapy, but hundreds of thousands undergo functional endoscopic sinus surgery (FESS) for refractory CRS every year. Patients with CRS often have a reduced quality of life, and may require billions of dollars in annual health-care and lost work time costs. CRS is a Th1 and Th2 inflammatory response to a number of mechanisms including but not limited to bacterial toxins, extracellular polysaccharide matrices secreted by bacteria and contained within a bacterial biofilm, fungi, developed allergic reactions to both bacteria and fungi (IgE) and auto immune disorders,. Bacteria associated with CRS and its accompanying inflammation include *Staphylococcus aureus, Pseudomonas aerugihosa, Streptococcus pneumonia, Haemophilus influenzae* and *Moraxella catarrhalis.* Biofilms containing one or more of these species and possibly also containing fungi may be a factor in the pathogenesis of CRS, see e.g., Ramadan et al., "Chronic rhinosinusitis and biofilms", Otolaryngol Head Neck Surg. 132:414-417 (2005) and Ferguson et al., "Demonstration of Biofilm in Human Bacterial Chronic Rhinosinusitis", Am J Rhinol, 5:19, pp. 452-57 (2005). The extracellular polysaccharide (EPS) matrix, the toxins produced by the bacterial colony, and the fungi that the bacterial biofilm may harbor may each be capable of inciting an inflammatory response from the host.

Adenoids (pharyngeal tonsils) and tonsils (palatine tonsils) are involved in a number of diseases of the ear, nose and throat including COME, RAOM, pediatric chronic sinusitis, tonsillitis, adenoiditis, pediatric obstructive sleep apnea (OSA), adult OSA and chronic strep throat. Lingual tonsils can also become infected and may cause a variety of problems. Treatment for these various diseases initially is normally carried out using oral medications or, in the case of OSA through the use of continuous positive airway pressure (CPAP). Failure of these therapies is often followed by surgical removal of the tonsils, adenoids or both tonsils and adenoids either because they harbor bacteria or obstruct anatomy. Complications related to surgical removal procedures include post-operative bleeding, dehydration, weight loss, peritonsillar abscess, torticilis (neck stiffness), regrowth of tissue, repeat surgery due to incomplete removal of tissue, continued COME or RAOM, continued OSA, and occasionally death. Post-operative treatment has traditionally been limited to dietary limitation, rinses, and use of oral antibiotics to prevent post-operative pain and infections.

The etiology and chronicity of at least COME, RAOM, cholesteatoma and CRS appear to be related to the presence of bacterial biofilms as well as their recalcitrance post-surgery.

### SUMMARY OF THE INVENTION

Patients, and their parents in the case of children, dislike infection recurrence and the possible need to undergo repeat or additional surgery. Although antibiotics may initially be administered at elevated dosages to address these problems, antibiotics have been shown to be ineffective against chronic infections that involve a bacterial biofilm and administering them can also promote drug resistance in the targeted and other bacterial species. It would be highly desirable to employ alternative treatments that permit a reduction or elimination in the amount of required antibiotics yet discourage recurrence of the treated condition. When the treated condition involves a bacterial biofilm on a tissue surface, it would be desirable to remove or disrupt the biofilm more effectively than is the case when saline irrigation is employed, so that remaining bacteria may more effectively be attacked by antibiotics or by the body's own natural defenses. It would also be desirable to at least temporarily seal or otherwise protect the thus-treated surface in order to repel bacterial adherence and biofilm reformation. It would also be desirable to do so while meeting biocompatibility requirements for contact with human tissue, and while using small dosages of administered materials and short periods of application. Our copending application Serial No. 11/739,508 filed April 24, 2007 describes a solvating system comprising a metal ion sequestering agent and surfactant and its use to disrupt bacterial biofilms within the middle or inner ear.

The present invention provides a medicament system which comprises:
(a) a solvating system comprising a metal ion sequestering agent and a surfactant that can detach, remove or otherwise disrupt at least a part of a biofilm attached or adhered to at least a portion of the middle or inner ear, to a surface within a nasal or sinus cavity, or to oral or pharyngeal tissue, and
(b) a polymeric film-forming medical sealant that can provide a protective layer over a site on which such a biofilm has been disrupted,
   for use in a method for treating bacterial ear, nose or throat conditions, which method comprises:
   (i) applying said solvating system to a treatment site comprising a bacterial biofilm attached or adhered to at least a portion of the middle or inner ear, to a surface within a nasal or sinus cavity, or to oral or pharyngeal tissue,
   (ii) detaching, removing or otherwise disrupting at least a part of the biofilm, and then
   (iii) applying said polymeric film-forming medical sealant to the treatment site to provide a protective layer that adheres to natural tissues at the site and resists detachment or other disruption until natural degradation or resorption of the sealant takes place.

The disclosed medicament system may be used for treatment or post-operative care of the middle or inner ear, and for rhinologic, oral or pharyngic treatment or post-operative care. The disclosed medicament system may also be used to treat maladies or chronic conditions including chronic otitis media with effusion, Recurrent Acute Otitis Media, cholesteatoma, chronic rhinosinusitis, pediatric chronic sinusitis, tonsillitis, adenoiditis, sleep apnea, chronic strep throat and other bacterial ear, sinus, oral cavity or throat conditions.

### BRIEF DESCRIPTION OF THE DRAWING

**Fig. 1** is a schematic cross-sectional view of a middle ear undergoing treatment via the disclosed method.
**Fig. 2** is an enlarged view of a portion of **Fig. 1****.**
**Fig. 3** is a schematic view showing the a sinus cavity undergoing treatment via the disclosed method;
**Fig. 4** is a perspective view of a surgical biofilm removal instrument which may be used in the disclosed method.

Like reference symbols in the various figures of the drawing indicate like elements. The elements in the drawing are not to scale.

### DETAILED DESCRIPTION OF THE PREFERRED EMBODIMENTS

The following detailed description describes certain embodiments and is not to be taken in a limiting sense. All weights, amounts and ratios herein are by weight, unless otherwise specifically noted. The terms shown below have the following meanings:

The term "antimicrobial agent" refers to a substance having the ability to cause greater than a 90% numeric reduction (viz., at least a 1-log order reduction) in a population of one or more of *Staphylococcus aureus, Pseudomonas aeruginosa, Streptococcus pneumonia, Haemophilus influenzae* or *Moraxella catarrhalis* or any other bacteria implicated in the etiology of COME, RAOM, cholesteatoma or chronic rhinosinusitis using the bacterial plate count procedure described below in the Examples.

The terms "attached" and "adhered" when used in reference to a bacterial biofilm and a surface mean that the biofilm is established on and at least partially coats or covers the surface, and has some resistance to removal from the surface. As the nature of this relationship is complex and poorly understood, no particular mechanism of attachment or adherence is intended by such usage.

The term "adhesion" refers to the sticking together of a material to tissues or tissue to tissue with which it is in intimate contact for extended periods or tissue that connects opposing tissues or prosthetic materials across a normally open space.

The term "bacterial biofilm" means a community of bacteria attached to a surface, with the organisms in the community being contained within an EPS matrix produced by the bacteria.

The term "biocompatible" when used in reference to a substance means that the substance presents no significant deleterious or untoward effects upon the body.

The term "biodegradable" when used in reference to a substance means that the substance will degrade or erode in vivo to form smaller chemical species. Such degradation process may be enzymatic, chemical or physical.

The term "bioresorbable" when used in reference to a substance means that the substance is capable of being absorbed by the body.

The terms "detaching", "removing" and "disrupting" when used in reference to a bacterial biofilm attached or adhered to a surface mean that at least a significant amount of the biofilm initially present on the surface no longer is attached or adhered to the surface. No particular mechanism of detachment, removal or disruption is intended by such usage.

The term "hemostat" means a device or material which stops blood flow.

The term "nasal or sinus cavities" refers to the various tissues defining the normally air-filled passages and chambers within the nose and sinus including but not limited to the nostrils or nares, the nasal concha or turbinates, the frontal, ethmoid, sphenoid and maxillary sinuses, the sinus ostia and the nasopharnyx, and to objects or articles (e.g., prostheses, packing or stents) that may be placed within a nasal or sinus cavity.

The term "polymeric sealant" means that the sealant is either formed from a synthetic crosslinked or uncrosslinked polymer or is a natural material such as a protein which has been crosslinked (e.g., synthetically crosslinked).

The term "residence time" when used in reference to a polymeric sealant at a treatment site means the time period during which the sealant remains in place *in vivo* under gross observation.

The term "sequestering agent" means a chemical that will combine with another material, especially a metal ion, to discourage or prevent the material from coming out of solution. The term "metal ion sequestering agent" means a sequestering agent that will combine with one or more metal ions such as alkali metals, alkaline earth metals, iron and the like to discourage or prevent the metal ion from coming out of solution. In order of increasing atomic number the alkali metals are lithium, sodium, potassium, rubidium, cesium, and francium, and the alkaline earth metals are beryllium, magnesium, calcium, strontium, barium, and radium.

The term "solvating" means to form a solution or dispersion containing a solvent or other carrier within which a solute is dissolved or suspended.

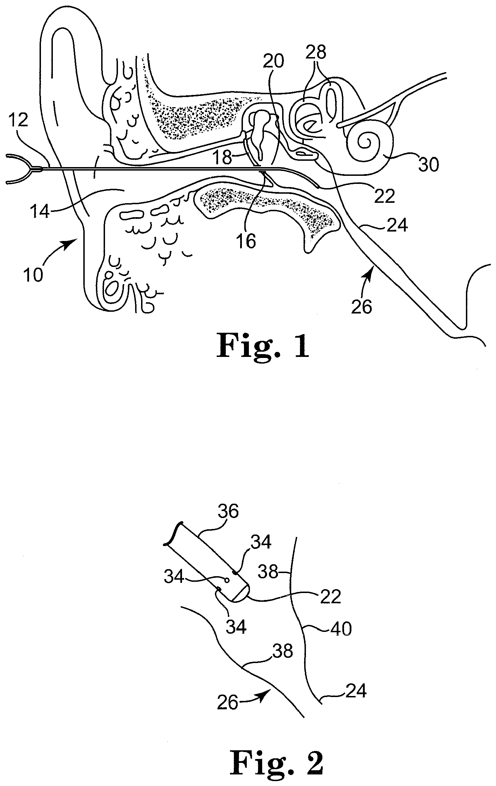

Referring to **Fig. 1****,** the disclosed method may be performed within ear **10** by inserting cannula **12** through ear canal **14** and ear tube **16** (which may for example have been installed via myringotomy) or other available opening in tympanic membrane **18** and thence into middle ear **20.** Cannula **12** may also be inserted in other ways without myringotomy, such as through a needle or other guidance device directed through the ear, Eustachian tubes, nose or mouth, and operated blindly or by using guided techniques such as microendoscopy, virtual image guided endoscopy, or image guided surgery using a flexible, tip tracked device. As shown in **Fig. 1****,** the distal end **22** of cannula **12** is positioned above isthmus **24** of Eustachian tube **26.** Cannula **12** may be positioned and if need be modified in shape or size so as to treat other portions of middle ear **20** (which for purposes of this discussion will be deemed to include at least the tympanic membrane, the lining of the middle ear, interior structures such as the ossicular chain and bordering structures such as the mastoid) or to treat portions of the inner ear (which for purposes of this discussion will be deemed to include at least semicircular canals **28** and cochlea **30**). For example, if treatment in the inner ear is desired, a further access opening (e.g., in a membrane near the round window or oval window) may be made.

**Fig. 2** shows an enlarged view of a portion of **Fig. 1****.** The solvating system is shown being applied to a bacterial biofilm proximate the Eustachian tube isthmus by dispensing the solvating system through orifices **34** located in sidewall **36** onto a bacterial biofilm such as biofilm **38** disposed on upper portion **40** of Eustachian tube **26.** Those skilled in the art will appreciate that the solvating system may be applied to a desired treatment site using methods or devices other than cannula **12.** Exemplary such methods include trephination and exemplary such devices include syringes (e.g., glass syringes and bulb syringes) and other devices suitable for providing access to the middle or inner ear via the tympanic membrane, Eustachian tubes or nose.

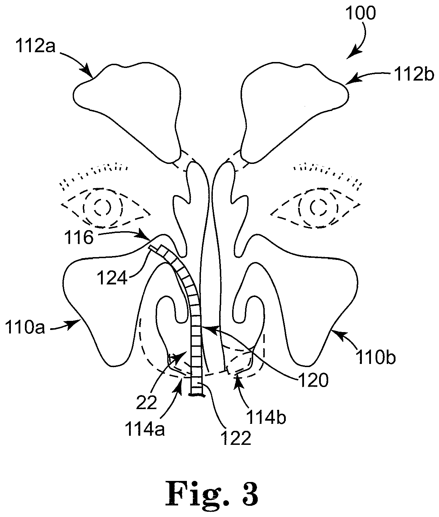

Referring to **Fig. 3****,** the disclosed method may be performed in the nasal or sinus cavities **100** of a patient, including the maxillary sinuses **110a**, **110b** and frontal sinuses **112a, 112b,** which may be accessed through nares **114a, 114b.** It should be noted that external features of the patient, including nares **114a, 114b,** are shown in dashed lines. When the patient suffers for example from chronic rhinosinusitis, one or more treatment sites such as treatment site **116** associated with a surface of maxillary sinus **110a** may be surgically addressed to substantially remove part or all of the biofilm at the treatment site and prevent or discourage bacterial recolonization. Treatment site **116** includes ciliated epithelium of maxillary sinus **110a** and an associated layer of bacteria inhabiting an associated biofilm (not shown in **Fig. 3**). The treatment site need not be natural tissue and may instead be an artificial structure (not shown in **Fig. 3**) such as a sinus packing or stent covered at least in part with a layer of bacterial biofilm. The disclosed solvating system may be applied treatment site **116** using an introducer **120** with an articulatable nozzle **122** containing an irrigation duct (hidden in **Fig. 3**) through which the solvating system may flow to the distal end of introducer and thence to the treatment site. The solvating system and residues of the biofilm may be removed from the treatment site via an aspiration duct (hidden in **Fig. 3**). The disclosed polymeric film-forming medial sealant may likewise be applied at the treatment site using the same or a different irrigation duct in introducer **120.** Those skilled in the art will appreciate that the solvating system, sealant or both solvating system and sealant may be applied to the treatment site using other methods or devices. Exemplary other methods include trephination and exemplary other devices include syringes (e.g., glass syringes and bulb syringes).

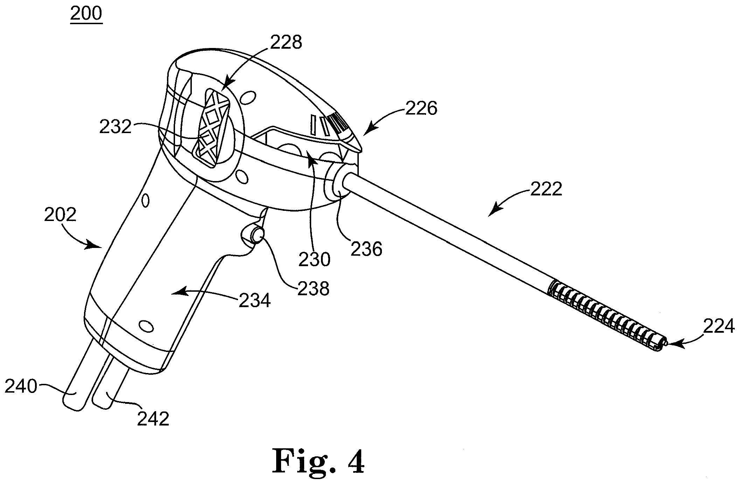

**Fig. 4** shows an exemplary biofilm removal surgical instrument **200** which may be used in the disclosed method. Instrument **200** includes a handle **202,** an introducer **222,** a nozzle **224** (referenced generally) and irrigation and aspiration ducts (not shown in **Fig. 4**). Instrument **200** can optionally further include a first actuator assembly **226** (referenced generally) and a second actuator assembly **228** (referenced generally). A control wheel **230** in first actuator assembly **226** may be operable by a user to effectuate bending of the introducer **222,** and a control wheel **232** in second actuator assembly **228** may be operable by a user to effectuate movement or rotation of nozzle 224 relative to introducer **222.** The handle **202** serves generally as a housing for various other components of instrument 200 and retains introducer **222.** Handle **202** may have a pistol grip-like shape, defining a grip portion **234** and a nose **236.** The grip portion **234** is sized and shaped for grasping by a user's hand, whereas the nose **236** is adapted for connection to the introducer **222.** Trigger **238** and an associated sensor and valve (not shown in **Fig. 4****)** may be used to control the flow of the disclosed solvating system through irrigation tubing **240** and thence to the distal end of introducer **222** through nozzle **224** and onto the desired treatment site. Trigger **238** may be provided with a multidirectional range of motion and associated with one or more additional sensors and valves (not shown in **Fig. 4**) to control removal of the solvating system, biofilm residue and other debris from the treatment site through nozzle **224** and thence to aspiration tubing **242.** Trigger **238** may also be used to control the flow of the disclosed sealant through a separate lumen in irrigation tubing **240** and thence to the distal end of introducer **222** through nozzle **224** and onto the desired treatment site.

The disclosed method may also be performed by applying the solvating system and sealant to the tonsils, adenoids or adjacent tissue. This may be done when the tonsils and adenoids are intact. The disclosed method may also or instead be performed postoperatively after removal of the tonsils, adenoids or both tonsils and adenoids, e.g., by applying the solvating system and sealant to the throat, such as to the tonsillar fossa.

The solvating system may be directed through a catheter, cannula, syringe, introducer or other appropriate conduit and applied to the desired treatment site to detach, remove or otherwise disrupt at least a part of a bacterial biofilm attached or adhered to the treatment site. The solvating system desirably is applied in at least an amount and thickness sufficient to cover the desired portion of the biofilm. The treatment may involve chemical dilution or mechanical disruption. For example, the solvating system may with appropriate care be applied as a pressurized spray to dislodge the bacterial biofilm, bacteria and other foreign body buildup at the treatment site. Application of the solvating system or other liquids (e.g., saline solution) may in appropriate treatment sites be performed using greater amounts of pressure, spray patterns, motion or other techniques to accomplish hydrodebridement at the treatment site. While not wishing to be bound by theory, the solvating system may dissolve the biofilm and bring it into solution or suspension so that the thus-disrupted biofilm can be easily flushed or otherwise removed from the treatment site using aspiration, lavage or other removal techniques. For example, in a procedure performed in the middle ear this may be accomplished via a myringotomy or through the Eustachian tube or nose. Any remaining bacteria at the treatment site may then more readily be attacked by an antimicrobial agent or by the body's natural defenses. Bacterial attack may for example be assisted by including an antimicrobial agent in the solvating system or in the polymeric film-forming medical sealant, or by separately applying an antimicrobial agent intra operatively or post operatively (e.g., topically, orally or systemically). It may be desirable to inject sufficient solvating system into the treatment area to displace any pus or other material that may be present, allowing excess material to overflow from the treatment area until the color of the excess material no longer changes. The solvating system may be left in place until it can drain away or is otherwise eliminated or resorbed, or the solvating system may be allowed to stand for a suitable time (e.g., a few minutes, a few hours or longer) and then may be rinsed away using saline or another suitable liquid. The solvating system preferably is applied directly to the treatment site, as such direct application may promote faster biofilm breakup. For example, for procedures performed in the middle or inner ear the solvating solution preferably is applied directly into the middle or inner ear region rather than merely being applied to the ear canal and allowed to transport across the tympanic membrane. Application of the solvating system and removal of dislodged or disrupted biofilm and bacteria may also be repeated as desired to ensure thorough removal of the offending organisms.

To discourage bacterial recolonization and biofilm reformation, the disclosed polymeric film-forming medical sealant is also applied to the treatment site. This may for example be accomplished using cannula **12** as shown in **Fig. 1** and **Fig. 2****,** introducer **120** or **222** as shown in **Fig. 3** and **Fig. 4****,** or a catheter, syringe or other appropriate conduit to dispense the film-forming medical sealant onto the treatment site. The applied sealant may fill the treatment site when applied but desirably does not do so and instead preferably is applied as a film or other conformal coating that leaves at least a partial air opening at the treatment site. For example, by applying only a coating rather than filling a middle ear the treatment site, the ossicular chain may remain free to move with movement of the tympanic membrane due to sound pressure waves and the function of the middle and inner ear may be preserved during healing. If desired, the sealant may be applied to part or all of the ossicular chain or to part or all of the tympanic membrane in order to provide a degree of stabilization during healing. As a further example, by applying only a coating rather than filling a nasal treatment site, breathing functions may be preserved during healing. The sealant desirably adheres to natural tissues at the treatment site and resists detachment or other disruption until natural degradation or resorption of the sealant takes place (e.g., after a residence time of one or more days, weeks or months). Meanwhile recolonization or reinfection may be significantly reduced or prevented, and improved healing and reciliation may take place. The sealant may provide various therapeutic advantages including but not limited to bacterial adhesion repellence, anti-infective properties, local immune modulation, tissue protection, reduction or elimination of pain or bleeding, reduction in inflammation, optimization of environment for ciliary regrowth, reduction in adhesions to critical anatomy, or the like. These advantages may arise due to a variety of mechanisms including a) inhibiting bacterial colonization, b) inhibiting the adherence of bacteria to tissue, c) reducing tissue morbidity or abscess formation, d) reducing or preventing disease recurrence (for example, specifically reducing the chronic inflammation related to bacterial toxin and EPS), e) coating and protecting tissue during healing, such as by maintenance of a moist wound which promotes platelet aggregation, or by closure of a dry wound without excessive scabrous formation, f) hemostasis, g) optimizing the environment for reciliation of the mucosa, h) speeding the growth or regrowth of cilia, i) preventing adhesion of a prosthesis or tympanomucosal grafts to native tissue, and j) delivering therapeutic agent(s) to the treatment site. Desirably the sealant will attach to a portion of the mucosa, cover other portions of the mucosa while leaving the cilia in such unattached portions free to undergo natural rhythmic cilia motion (viz., cilia beating), deliver antimicrobial agents or additional therapeutic agents as needed, and prevent bacteria from adhering to the treatment site.

A variety of solvating systems may be used in the disclosed method. As noted above, the solvating system comprises surfactant. The surfactant desirably is water-soluble and nontoxic. Exemplary surfactants include anionic surfactants, nonionic surfactants, cationic surfactants and zwitterionic surfactants. Exemplary anionic surfactants include but are not limited to C₆-C₂₄ alkylbenzene sulfonates; C₆-C₂₄ olefin sulfonates; C₆-C₂₄ paraffin sulfonates; cumene sulfonate; xylene sulfonate; C₆-C₂₄ alkyl naphthalene sulfonates; C₆-C₂₄ alkyl or dialkyl diphenyl ether sulfonates or disulfonates, C₄-C₂₄ mono or dialkyl sulfosuccinates; sulfonated or sulfated fatty acids; C₆-C₂₄ alcohol sulfates (for example C₆-C₁₂ alcohol sulfates); C₆-C₂₄ alcohol ether sulfates having 1 to about 20 ethylene oxide groups; C₄-C₂₄ alkyl, aryl or alkaryl phosphate esters or their alkoxylated analogues having 1 to about 40 ethylene, propylene or butylene oxide units; and mixtures thereof. For example, the anionic surfactant may be sodium chenodeoxycholate, N-lauroylsancosine sodium salt, lithium dodecyl sulfate, 1-octanesulfonic acid sodium salt, sodium cholate hydrate, sodium deoxycholate, sodium dodecyl sulfate (also known as sodium lauryl sulfate) or sodium glycodeoxycholate.

Exemplary cationic surfactants include but are not limited to quaternary amine compounds having the formula: 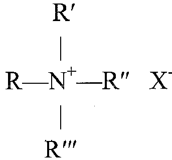 where R, R', R" and R"' are each a C₁-C₂₄ alkyl, aryl or aralkyl group that can optionally contain one or more P, O, S or N heteroatoms, and X is F, Cl, Br, I or an alkyl sulfate. For example, the cationic surfactant may be hexadecylpyridinium chloride monohydrate or hexadecyltrimethylammonium bromide.

Exemplary nonionic surfactants include but are not limited to C₆-C₂₄ alcohol ethoxylates (for example C₆-C₁₄ alcohol ethoxylates) having 1 to about 20 ethylene oxide groups (for example about 9 to about 20 ethylene oxide groups); C₆-C₂₄ alkylphenol ethoxylates (for example C₈-C₁₀ alkylphenol ethoxylates) having 1 to about 100 ethylene oxide groups (for example about 12 to about 20 ethylene oxide groups); C₆-C₂₄ alkylpolyglycosides (for example C₆-C₂₀ alkylpolyglycosides) having 1 to about 20 glycoside groups (for example about 9 to about 20 glycoside groups); C₆-C₂₄ fatty acid ester ethoxylates, propoxylates or glycerides; C4-C₂₄ mono or di alkanolamides; and mixtures thereof. For example, the nonionic surfactant may be polyoxyethyleneglycol dodecyl ether, N-decanoyl-N-methylglucamine, digitonin, n-dodecyl B-D-maltoside, octyl B-D-glucopyranoside, octylphenol ethoxylate, polyoxyethylene (8) isooctyl phenyl ether, polyoxyethylene sorbitan monolaurate or polyoxyethylene (20) sorbitan monooleate.

Exemplary zwitterionic surfactants include but are not limited to aminoalkylsulfonate compounds having the formula: 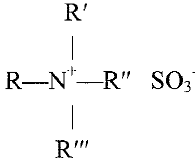 where R, R', R" and R"' are each a C₁-C₂₄ alkyl, aryl or aralkyl group that can optionally contain one or more P, O, S or N heteroatoms; amine oxide compounds having the formula: 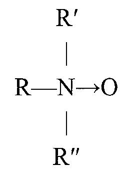 where R, R' and R" are each a C₁-C₂₄ alkyl, aryl or aralkyl group that can optionally contain one or more P, O, S or N heteroatoms; and betaine compounds having the formula: 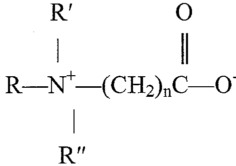 where R, R' and R" are each a C₁-C₂₄ alkyl, aryl or aralkyl group that can optionally contain one or more P, O, S or N heteroatoms, and n is about 1 to about 10. For example, the zwitterionic surfactant maybe 3-[(3-cholamidopropyl) dimethylammonio]-2-hydroxy-1-propane sulfonate, 3-[(3-cholamidopropyl) dimethylammonio]-1-propane sulfonate (sometimes referred to as CHAPS), 3-(decyldimethylammonio) propanesulfonate inner salt (sometimes referred to as caprylyl sulfobetaine), or N-dodecyl-N,N-dimethyl-3-ammonio-1-propanesulfonate.

Preferred surfactants include alkyl sulfates, alkyl sulfonates, aryl sulfonates and zwitterionic surfactants. The desired surfactants may be obtained as pure compounds or in some instances may be obtained by using products such as liquid Castile soap. The surfactant may for example be present at a concentration of at least about 0.002 M, at least about 0.005 M or at least about 0.01 M, e.g., about 0.002 to about 1 M, about 0.005 to about 0.7 M or about 0.01 to about 0.5 M. Expressed on a weight basis, the surfactant may for example be greater than 0.2 wt. % of the solvating system, e.g., about 0.3% to about 30%, about 0.5% to about 25% or about 1% to about 20% of the solvating system. Increased surfactant amounts may promote faster biofilm breakup.

The solvating system contains a metal ion sequestering agent. The sequestering agent desirably is a mild acid whose acidity is sufficient to sequester one or more metal ions in the exopolysaccharide or extracellular polysaccharide matrix, but which is not so acidic so as to harm the treated tissue. Metal ions of particular interest (due to their likely involvement in the targeted bacterial biofilms) include sodium, calcium and iron. The metal ion sequestering agent desirably is water-soluble, nontoxic and if used in the ear not prone to aggravate long-term hearing loss. Representative acids include but are not limited to carboxylic acids, diacids, or triacids such as formic acid, acetic acid, chloroacetic acid, dichloroacetic acid, oxalic acid, oxamic acid, glycolic acid, lactic acid, pyruvic acid, aspartic acid, fumaric acid, maleic acid, succinic acid, iminodiacetic acid, glutaric acid, 2-ketoglutaric acid, glutamic acid, adipic acid, citric acid, glucuronic acid, mucic acid, nitrilotriacetic acid, salicylic acid, ketopimelic acid, benzoic acid, mandelic acid, chloromandelic acid, phenylacetic acid, phthalic acid and boric acid; mineral acids such as hydrochloric acid, orthophosphoric acid and phosphonic acid; and mixtures thereof. Citric acid is a preferred acid. The metal ion sequestering agent may for example be present at a concentration of at least about 0.01 M, at least about 0.05 M or at least about 0.1 M, e.g., about 0.01 to about 0.5 M, about 0.05 to about 0.4 M or about 0.1 to about 0.3 M. Increased metal ion sequestering agent amounts may promote faster biofilm breakup.

The solvating system may optionally include a variety of other ingredients, including water and other solvents (e.g., alcohols), buffering agents, antimicrobial agents and a variety of adjuvants. Preferably the solvating system contains water and one or more buffering agents. The buffering agent preferably maintains the solvating system at an appropriate pH for contacting human tissue, and desirably at a pH greater than 5. For example, the solvating system may be buffered to have a near-neutral pH, e.g., a pH greater than 5 and less than 8.5. Buffering agents may for example be up to about 25% of the solvating system. Exemplary buffering agents include but are not limited to potassium chloride, glycine, potassium hydrogen phthalate, sodium acetate, potassium hydrogen phthalate, barbitone sodium and sodium citrate. When the metal ion sequestering agent is a mild acid, the buffering agent desirably is a salt of that acid.

Solvating systems containing one or more antimicrobial agents are also preferred. The EPS matrix allows the biofilm to stick to an underlying surface and also protects the embedded organisms; thus, bacteria in biofilms are approximately 100 to 1000 times more resistant to the effects of antibiotics than planktonic bacteria. After the biofilm has been broken down into unbound polymers or fragments and solvated or otherwise disrupted by the solvating system, an antimicrobial agent can much more effectively attack the remaining bacteria. Exemplary antimicrobial agents include active oxygen compounds such as hydrogen peroxide, isolated or equilibrium derived or isolated peracids such as chloroperbenzoic acids, peracetic acid, perheptanoic acid, peroctanoic acid, perdecanoic acid, performic acid, percitric acid, perglycolic acid, perlactic acid, perbenzoic acid, and monoester peracids derived from diacids or diesters such as adipic, succinic, glutaric, or malonic acid; aminoglycosides; amphenicols; ampicillins; ansamycins; beta-lactams such as carbacephems, carbapenems, cephalosporins, cephamycins, monobactams, oxacephems, penicillins and any of their derivatives; carboxylic esters such as p-hydroxy alkyl benzoates and alkyl cinnamates; chitosan salts; cubic-phase lipids; gallium-containing antimicrobial agents such as gallium acetylacetonate, gallium bromide, gallium chloride, gallium fluoride, gallium iodide, gallium maltolate, gallium nitrate, gallium nitride, gallium percolate, gallium phosphide and gallium sulfate; iodo-compounds and other active halogen compounds such as iodine, interhalides, polyhalides, metal hypochlorites, hypochlorous acid, metal hypobromites, hypobromous acid, chloro- and bromo-hydantoins, chlorine dioxide and sodium chlorite; lincosamides; macrolides; nitrofurans; organic peroxides including benzoyl peroxide and alkyl benzoyl peroxides; ozone; phenolic derivatives including o-phenyl phenol, o-benzyl-p-chlorophenol, tert-amyl phenol and C₁-C₆ alkyl hydroxy benzoates; quaternary ammonium compounds such as alkyldimethylbenzyl ammonium chloride and dialkyldimethyl ammonium chloride; quinolines; singlet oxygen generators; sulfonamides; sulfones; sulfonic acids such as dodecylbenzene sulfonic acid; tetracycline antibiotics such as tetracycline, chlonetracycline, oxytetracycline, demecocycline, doxycycline, lymecycline, mecloclycline, methacycline, methocycline, minocycline, and the like; vancomycin; derivatives thereof and mixtures thereof. Many of these recited agents represent classes containing useful specific materials whose individual utility will be recognized by persons having ordinary skill in the art. For example, exemplary penicillins include but are not limited to amdinocillin, amdinocillin pivoxil, amoxicillin ampicillin, apalcillin, aspoxicillin, axidocillin, azlocillin, acampicillin, bacampicillin, benzylpenicillinic acid, benzylpenicillin sodium, carbenicillin, carindacillin, clometocillin, cloxacillin, cyclacillin, dicloxacillin, epicillin, fenbenicillin, floxacillin, hetacillin, lenampicillin, metampicillin, methicillin sodium, mezlocillin, nafcillin sodium, oxacillin, penamecillin, penethamate hydriodide, penicillin G benethamine, penicillin G benzathine, penicillin G benzhydrylamine, penicillin G calcium, penicillin G hydrabamine, penicillin G potassium, penicillin G. procaine, penicillin N, penicillin O, penicillin V, penicillin V banzathine, penicillin V hydrabamine, penimepicycline, phenethicillin potassium, piperacillin, pivampicillin propicillin, quinacillin, sulbenicillin, sultamicillin, talampicillin, temocillin, ticarcillin and mixtures thereof or with other materials (e.g., penicillins combined with clavulanic aid such as the combination of amoxicillin and clavulanic acid available as AUGMENTIN™ from GlaxoSmithKline).

An antimicrobial agent such as those described above may optionally be applied in a separate treatment step (if need be in a suitable carrier) after application of the solvating system and before application of the polymeric film-forming medical sealant. An antimicrobial agent may also be applied as a part of the sealant. Whether applied as a part of the solvating system, in a separate step, or as a part of the sealant, the antimicrobial agent preferably provides greater than a 99% numeric reduction (*viz*., at least a 2-log order reduction), greater than a 99.9% numeric reduction (*viz*., at least a 3-log order reduction), greater than a 99.99% numeric reduction (viz., at least a 4-log order reduction) or greater than a 99.999% numeric reduction (viz., at least a 5-log order reduction) in a population of one or more of *S. aureus, P. aeruginosa, S. pneumonia, H. influenzae* or *M. catarrhalis* bacteria using the bacterial plate count procedure described below in the Examples.

The solvating system may contain additional therapeutic agents. Exemplary therapeutic agents include any material suitable for otologic, rhinologic or pharyngic use including analgesics, anti-cholinergics, anti-fungal agents, antihistamines, blood products, steroidal or non-steroidal anti-inflammatory agents, anti-parasitic agents, antiviral agents, biostatic compositions, chemothenapeutic/antineoplastic agents, cytokines, decongestants, immunosuppressors, mucolytics, nucleic acids, peptides, proteins, steroids, vasoconstrictors, vitamins, mixtures thereof, and other therapeutic materials that will be apparent to those skilled in the art. Several such additional therapeutic agents are discussed in more detail below in connection with the polymeric film-forming medical sealant. Other adjuvants that may be included in the solvating system include dyes, pigments or other colorants (e.g., FD & C Red No. 3, FD & C Red No. 20, FD & C Yellow No. 6, FD & C Blue No.2, D & C Green No. 5, D & C Orange No. 4, D & C Red No. 8, caramel, titanium dioxide, fruit or vegetable colorants such as beet powder or beta-carotene, turmeric, paprika and other materials that will be familiar to those skilled in the art); indicators; flavoring or sweetening agents including but not limited to anise oil, cherry, cinnamon oil, citrus oil (e.g., lemon, lime or orange oil), cocoa, eucalyptus, herbal aromatics (e.g., clove oil, sage oil or cassia oil), lactose, maltose, menthol, peppermint oil, saccharine, sodium cyclamate, spearmint oil, sorbitol, sucrose, vanillin, wintergreen oil, xylitol and mixtures thereof; antioxidants; antifoam agents; and rheology modifiers including thickeners and thixotropes.

The solvating system desirably has a sufficiently low viscosity to enable easy delivery to the treatment site using for example power spray or other spray application, lavage, misting, mopping, wicking or dripping. The solvating system desirably also may be easily removed from the treatment site by subsequent flushing, rinsing, draining or absorption. The solvating system need not be applied in liquid form and may for example be applied as a powder, gel, foam, sponge, film strip or other suitable form. The solvating system may be applied to treatment sites in the middle or inner ear, to associated structures such as the Eustachian tubes, to the nasal or sinus cavities or to oral or pharyngeal tissues. The solvating system is especially well-suited to the treatment of ciliated tissues. The solvating system preferably is biocompatible with the delicate tissues and structures of the middle or inner ear, and desirably does not contain ingredients which might potentially harm such tissues or structures or unduly compromise long-term hearing.

A variety of polymeric film-forming medical sealants may be used in the disclosed method. The sealant preferably is a biodegradable or bioresorbable material having a residence time in vivo of from one day to a few (e.g., 2, 3 or 4) days, weeks or months. The sealant may be uncrosslinked, crosslinked before being applied to the treatment site, or crosslinked after application. In one embodiment, the sealant may be a viscoelastic material. In another embodiment, the sealant may harden after application. The sealant may be a synthetic polymer (for example, polyethylene glycol or PEG), natural polymer (for example, a polysaccharide, lipid or polypeptide), or a synthetically-modified natural polymer (for example, a polypeptide reacted with PEG). Other exemplary synthetic polymers include polyacetals, polyacrylic acid, polyalkylene oxalates, polyalkylene succinates, polyamides, polyamino acids, polyaspartic acid, polyanhydrides, polycaprolactones, polycarbonates, polycyanoacrylates, polydiaxonones, polyesteramides, polyetheresters, polyethylene oxide (PEO), poly(glycolic acids) and other poly(glycolides), polyhydroxybutyrates, polyhydroxyvalerates, polyketals, poly(lactic acid) and other polylactides including poly(lactide-co-glycolides), poly(malic acids), polyorthoestei's, polyphosphazines, polyphosphoesters, polypropylene oxide (PPO), degradable polyurethanes, polyvinyl alcohol (PVA), polyvinyl pyrrolidone (PVP), and copolymers, terpolymers, blends, and mixtures thereof.

Exemplary polysaccharides include agar; cellulose and its derivatives such as oxidized cellulose, hydroxyethyl cellulose, carboxymethyl cellulose (CMC), carboxymethyl amylose (CMA), carboxyethyl cellulose and hydroxypropylmethyl cellulose (HPMC); chitin; chitosan and its derivatives such as carboxymethyl chitosan and trimethylchitosan; dextran and its derivatives such as carboxymethyl dextran; glycogen; glycosaminoglycans such as hyaluronan (e.g., hyaluronic acid and its derivatives including esters and polymers), heparin, heparin sulfate, dermatin sulfate, and chondroitin-6-sulfate; gums such as alginate, gellan gum and xanthan gum; pectin; and starch and its derivatives.

Exemplary lipids include glyceryl based lipid compounds such as glyceryl monooleate, and liquid crystal lipids which can be delivered in fluid form and which when in contact with moisture will convert to a cubic phase to provide a waxy cubic or crystalline material.

Exemplary polypeptides include albumin, collagen, gelatin, silk and their derivatives. For example, crosslinked hydrogels may be formed from many polypeptides by reacting them with a suitable crosslinking agent such as an aldehyde (e.g., glutaraldehyde or formaldehyde), carbodiimide, chitin, CMC, diisocyanate, or a glycol such as a PEG.

The polymeric film-forming medical sealant may include antimicrobial agents, additional therapeutic agents and other adjuvants like those mentioned above in connection with the solvating system. Sealants containing therapeutic agents that offer both anti-infective and anti-inflammatory properties (e.g., tetracyclines) are a preferred embodiment. Sealants containing additional therapeutic agents such as anti-fungal agents, antihistamines, steroidal or non-steroidal anti-inflammatory agents, anti-parasitic agents, antiviral agents, chemotherapeutic/antineoplastic agents, decongestants or mucolytics are another preferred embodiment. Sealants containing antimicrobial agents and additional therapeutic agents are yet another preferred embodiment. Exemplary anti-fungal agents include but are not limited to allylamines, imidazoles, polyenes, thiocarbamates, triazoles, derivatives thereof and mixtures thereof. Exemplary antihistamines include but are not limited to azelastine, diphenhydramine, loratidine, derivatives thereof and mixtures thereof. Exemplary steroidal anti-inflammatory agents include but are not limited to 21-acetoxypregnenolone, alclometasone, algestone, amcinonide, beclomethasone, betamethasone, budesonide, chloroprednisone, clobetasol, clobetansone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethosone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, derivatives thereof and mixtures thereof. Preferred steroidal anti-inflammatory agents include beclomethasone, budesonide, fluticasone proprionate and mometasonefuroate. Exemplary nonsteroidal anti-inflammatory agents include but are not limited to COX inhibitors (COX-1 or COX nonspecific inhibitors) and selective COX-2 inhibitors. Exemplary COX inhibitors include but are not limited to salicylic acid derivatives such as aspirin, sodium salicylate, choline magnesium trisalicylate, salicylate, diflunisal, sulfasalazine and olsalazine; para-aminophenol derivatives such as acetaminophen; indole and indene acetic acids such as indomethacin and sulindac; heteroaryl acetic acids such as tolmetin, dicofenac and ketorolac; arylpropionic acids such as ibuprofen, naproxen, flurbiprofen, ketoprofen, fenoprofen and oxaprozin; anthranilic acids (fenamates) such as mefenamic acid and meloxicam; enolic acids such as the oxicams (piroxicam, meloxicam); alkanones such as nabumetone; derivatives thereof and mixtures thereof. Exemplary COX-2 inhibitors include but are not limited to diaryl-substituted furanones such as refecoxib; diaryl-substituted pyrazoles such as celecoxib; indole acetic acids such as etodolac and sulfonanilides such as nimesulide; derivatives thereof and mixtures thereof. Exemplary anti-parasitic agents include but are not limited to atovaquone clindamycin, dapsone, iodoquinol, metronidazle, pentamidine, primaquine, pyrimethamine, sulfadiazine, trimethoprim/sufamethoxazole, trimetrexate, derivatives thereof and mixtures thereof. Exemplary antiviral agents include but are not limited to acyclovir, famciclovir, valacyclovir, edoxudine, ganciclovir, foscamet, cidovir (available as VISTIDE™ from Gilead Sciences, Inc.), vitrasert, formivirsen, HPMPA (9-(3-hydroxy-2-phosphonomethoxypropyl)adenine), PMEA (9-(2- phosphonomethoxyethyl)adenine), HPMPG (9-(3-hydroxy-2-(phosphonomethoxy)propyl)guanine), PMEG (9-[2-(phosphonomethoxy)ethyl]guanine), HPMPC (1-(2-phosphonomethoxy-3-hydroxypropyl)-cytosine), ribavirin, EICAR (5-ethynl-1-beta-D-ribofuranosylimidazole-4-carbonxamine), pyrazofurin (3-[beta-D-ribofuranosyl]-4-hydroxypyrazole-5-carboxamine), 3-Deazaguanine, GR-92938X (1-beta-D-ribofuranosylpyrazole-3, 4-dicarboxamide), LY253963 (1,3,4-thiadiazol-2-yl-cyanamide), RD3-0028 (1,4-dihydro-2,3-benzodithiin), CL387626 (4,4'-bis[4,6-di][3-aminophenyl-N,N-bis(2-carbamoylethyl)-sulfonilimino]-1,3,5-triazine-2-ylamino-biphenyl-2-,2'-disulfonic acid disodium salt), BABIM (bis[5-amidino-2-benzimidazoly-1]-methane), NIH351, derivatives thereof and mixtures thereof. Exemplary chemotherapeutic/antineoplastic agents include but are not limited to antitumor agents (e.g., cancer chemotherapeutic agents, biological response modifiers, vascularization inhibitors, hormone receptor blocks, and cryotherapeutic agents or other agents that destroy or inhibit neoplasia or tumorigenesis) such as alkylating agents or other agents which directly kill cancer cells by attacking their DNA (e.g., cyclophosphamide and isophosphamide), nitrosoureas or other agents which kill cancer cells by inhibiting changes necessary for cellular DNA repair (e.g., carmustine (BCNU) and lomustine (CCNU)), antimetabolites and other agents that block cancer cell growth by interfering with certain cell functions, usually DNA synthesis (e.g., 6 mercaptopurine and 5-fluorouracil (5FU)), antitumor antibiotics and other compounds that act by binding or intercalating DNA and preventing RNA synthesis (e.g., doxorubicin, daunorubicin, epirubicin, idarubicin, mitomycin-C and bleomycin), plant (vinca) alkaloids and other anti-tumor agents derived from plants (e.g., vincristine and vinblastine), steroid hormones, hormone inhibitors, hormone receptor antagonists and other agents which affect the growth of hormone-responsive cancers (e.g., tamoxifen, herceptin, aromatase inhibitors such as aminoglutethamide and formestane, triazole inhibitors such as letrozole and anastrazole, and steroidal inhibitors such as exemastane), antiangiogenic proteins, small molecules, gene therapies or other agents that inhibit angiogenesis or vascularization of tumors (e.g., meth-1, meth-2 and thalidomide), bevacizumab (available as AVASTIN™ from Genentech), squalamine, endostatin, angiostatin, ANGIOZYME™ from Ribozyme Pharmaceuticals, neovastat (available as AE-941™ from Aeterna Zentaris), CC-5013 (available as REVIMID™ from Celgene Corp.), medi-522 (available as VITAXIN™ from MedImmune, Inc.), 2-methoxyestradiol or 2ME2 (available as PANZEM™ from Entremed, Inc.), carboxyamidotriazole (CAI), combretastatin A4 prodrug (CA4P), SU6668, SU11248, BMS-275291, COL-3, EMD 121974, IMC-1C11, IM862, TNP-470, celecoxib (available as CELEBREX™ from Pfizer Inc.), refecoxib, interferon alpha, interleukin-12 (IL-12) or any of the compounds identified in Science Vol. 289, Pages 1197-1201 (Aug. 17, 2000), biological response modifiers (e.g., interferon, bacillus calmette-guerin (BCG), monoclonal antibodies, interluken 2, granulocyte colony stimulating factor (GCSF), etc.), PGDF receptor antagonists, herceptin, asparaginase, busulphan, carboplatin, cisplatin, carmustine, chlorambucil, cytarabine, dacarbazine, etoposide, flucarbazine, flurouracil, gemcitabine, hydroxyurea, ifosphamide, irinotecan, lomustine, melphalan, mercaptopurine, methotrexate, thioguanine, thiotepa, tomudex, topotecan, treosulfan, vinblastine, vincristine, mitoazitrone, oxaliplatin, procarbazine streptocin, taxol or paclitaxel, taxotere, analogs/congeners, derivatives thereof and mixtures thereof. Exemplary decongestants include but are not limited to epinephrine, oxymetazoline, phenylephrine, pseudoephedrine, tetrahydrozolidine, xylometazoline, derivatives thereof and mixtures thereof. Exemplary mucolytics include but are not limited to acetylcysteine, dornase alpha, guaifenesin, derivatives thereof and mixtures thereof.

In those instances where it is desirable to remove water from tissue, e.g., to remove fluid from polyps or edematous tissue, a hyperosmolar agent may be employed in the sealant. Exemplary hyperosmolar agents include but are not limited to furosemide, sodium chloride gel and other salt preparations that draw water from tissue or substances that directly or indirectly change the osmolar content of the mucous layer. Where sustained release or delayed release of the therapeutic agent is desirable, a release agent modifier may also be included in the sealant. The therapeutic agent may also be encapsulated, e.g., within polymeric microspheres, to further delay or sustain release of the therapeutic agent.

The sealant desirably has a sufficiently low viscosity to enable easy delivery to the treatment site using for example power spray or other spray application, lavage, misting, mopping, wicking or dripping. The sealant need not be applied in liquid form and may for example be applied as a powder, gel, foam, sponge, film strip or other suitable form. The sealant may also be a flowable liquid that crosslinks, polymerizes or otherwise alters its consistency to form a sealant. The sealant may be applied to treatment sites in the middle or inner ear, to associated structures such as the Eustachian tubes, to the nasal or sinus cavities or to oral or pharyngeal tissues. The sealant is especially well-suited for use on ciliated tissues. The applied sealant may be bioresorbable or biodegradable after a desired period of time once healing has occurred. The sealant desirably includes at least one characteristic that promotes retention of the sealant at the treatment site. This characteristic may be selected from a variety of features including but not limited to thickness, size, shape, density, viscosity, hardness, bioadhesiveness, mucoadhesiveness, manner of application or insertion, and the like. The sealant may prevent bacterial recolonization or the formation or reformation of bacterial biofilms by covering the treatment site (e.g., mucosa from which a bacterial biofilm has been removed by the solvating system) with an alternative film structure whose surface is not readily penetrable by bacteria associated with bacterial ear, nose or throat conditions. The sealant preferably is biocompatible with the delicate tissues and structures of the middle or inner ear, and desirably does not contain ingredients which might potentially harm such tissues or structures or unduly compromise long-term hearing.

The solvating system and sealant are used as a part of a multi-step treatment regimen which disrupts the bacterial biofilm and discourages its return. For example, a series of steps that may be broadly classified as Cleansing/Disrupting, Killing, Protecting/Coating, Aerating, and Healing may be carried out. The Cleansing/Disrupting step may be carried out by administering the solvating system as described above. The Killing step may be carried out by applying a suitable antimicrobial agent to the treatment site. This may as described above be accomplished by including an antimicrobial agent in the solvating system, in the sealant, or in both the solvating system and sealant. As noted above, an antimicrobial agent may also be applied as a separate step between application of the solvating system and application of the sealant. An antimicrobial agent may also be applied or administered post operatively. The Protecting/Coating step may be carried out by coating at least part of the thus-treated tissue with a protective sealant layer as described above. The Aerating step may be carried out by preserving or forming a suitable opening or openings (e.g., a slit in the tympanic membrane, or an opening in occluded or partially occluded nasal passages, sinuses or sinus ostia) and leaving it or them open for a period of time sufficient to allow aeration of the treatment site. The time period may be affected by the nature of the opening(s) and for ear treatments by whether or not a tympanostomy tube is installed. For example, if a slit has been formed in the tympanic membrane and a tube is not placed in the opening then the slit may remain open for a few days and heal over, thereby closing the ear space naturally. The Healing step may be carried out by allowing the cleansed, protected and sealed tissue surface to undergo a return to a normal state, e.g., through one or more healing mechanisms such as modulation of an inflammatory response, phagocytosis, mucosal remodeling, reciliation or full or partial restoration of normal hearing, balance or breathing. The disclosed method may in some instances advantageously be accomplished without requiring surgery, for example in nasal treatment by applying and removing the solvating system and by applying the sealant through normal aspiration/suction techniques or by simple flushing of affected nasal passages without infusing the solvating system or sealant into the more difficult to reach sinuses beyond the sinus ostia.

The invention is further illustrated in the following non-limiting examples.

### Example 1

Bacterial isolates of *S. aureus* and *P. aeruginosa* bacteria were recovered from the sinuses of patients with sinus disorders. Patients with cystic fibrosis or an underlying immunosuppressive disease (HIV infection, insulin-dependent diabetes mellitus, or renal disease) and patients who had taken antibiotics or oral prednisone in the previous month were excluded. All patients had refractory sinusitis, that is, persistent symptoms resistant to medical therapy despite having undergone technically successful functional endoscopic sinus surgery (FESS) for refractory chronic rhinosinusitis (CRS) with or without nasal polyposis. The occurrence of CRS was diagnosed in accordance with the 2003 American Academy of Otolaryngology-Head and Neck Surgery (AAO-HNS) guidelines set out in Benninger et al., "Adult chronic rhinosinusitis: Definitions, diagnosis, epidemiology, and pathophysiology", Otolaryngol Head Neck Surg 129(3 suppl):S1-S32 (2003). The selected patients had been refractory to medical therapy for more than 12 months before sample collection, and the failure of FESS was judged not to be associated with technical factors such as obstructive synechiae, frontal sinus obstruction, or a retained uncinate process. Samples were collected consecutively until 10 specimens each of *S. aureus* and *P. aeruginosa* were obtained using direct endoscopic guidance and the procedure described by Nadel et al., "Endoscopically guided cultures in chronic sinusitis", Am J Rhinol 12:233-241 (1998). Briefly, a topical anesthetic agent was administered, the nasal ala retracted, and an endoscope used to visualize the middle meatus and sinus cavities. A thin, flexible calcium alginate swab (STARSWAB II™ Collection and Transport System, Starplex Scientific, Etobicoke, Ontario) was inserted and directed to the site with the most purulence. If no purulence was observed, the surface of the maxillary sinus was swabbed for 15 seconds. Care was taken to avoid contact with the lateral nasal wall or nasal vestibule. Samples were plated and incubated using standard procedures. Bacteria were identified using a VITEK 2™ system (Biomérieux, Durham, NC). Crystal violet staining to confirm the presence of biofilms was performed according to the method described by Stepanovic et al., "A modified microtiter-plate test for quantification of staphylococcal biofilm formation", J Microbiol Methods 40:175-179 (2000). For incubation and culture, previously frozen strains were inoculated on trypticase soy agar (TSA) with 0.5% sheep blood. After 24 hours, one to four colonies per strain were cultured on TSA. Cultures were incubated at 37°C for 24 hours to condition them to a trypticase soy broth (TSB)-TSA medium and ensure noncontamination. Colonies grown on TSA solid medium were then amplified in 5 mL of TSB medium with 0.5% glucose according to the method described by Gotz, "Staphylococcus and biofilms", Mol Microbial 43:1367-1378 (2002) and incubated at 37°C for at least 24 hours.

A drip-flow reactor (DFR) was used to determine the effectiveness of various test solutions delivered to *S aureus* and *P aeruginosa* biofilms on hydroxyapatite (HA)-coated microscope slides for removing these bacterial biofilms with and without hydrodynamic force. The slides in the DFR are tipped at 10° from the horizontal, thereby modeling a low shear environment. The DFR was housed in an incubator at 37°C under aerobic conditions. Approximately 20 minutes before bacterial inoculation, sterile medium (10% TSB for *S aureus*; 1% TSB for *P aeruginosa*) was dripped on the slides in the DFR and allowed to collect over the slides to form a conditioning layer. The slides were then inoculated with 1 mL of a culture of either *S aureus* or *P aeruginosa.* The DFR was tilted so that the slides would be horizontal for 4 hours to allow bacterial attachment to the substrate. Subsequently, the DFR was set so that the slides were once again at a 10° angle, with sterile medium dripping on the slides at a rate of 10 mL per hour. After 3 days, biofilm-removal experiments were performed. Two methods were used to treat the biofilms formed by each bacterial species. The first application method involved a static treatment in the DFR, with a solvating agent (referred to as CAZS) being dripped onto the biofilms. The CAZS solvating agent contained deionized water, 25 g/L (corresponding to 0.13 M) citric acid, 5.35 g/L (corresponding to 0.02 M) caprylyl sulfobetaine zwitterionic surfactant (CH₃(CH₂)₉N⁺(CH₃)₂CH₂CH₂CH₂SO₃⁻, CAS 15163-36-7) and sufficient sodium citrate (about 240 g/L) to buffer the system to pH 5.4. The second application method involved delivery of saline or delivery of CAZS outside the DFR, using a pressurized jet lavage to apply a hydrodynamic shearing force to the biofilm. For all treatments, preliminary runs were done to ensure that variations among slides were within acceptable limits. In addition, multiple plates of both bacterial species were produced to determine the within-run and run-to-run variations. A control slide was made for each DFR run. Three runs were evaluated for each treatment of each type of bacteria.

For static treatment, flow to the DFR was halted, the DFR was placed in a horizontal position, and the cover was removed. A 25 mL portion of CAZS was applied to one slide. Control slides were not treated with CAZS. After 10 minutes, the slides were rinsed with saline (25 mL). The DFR was then disconnected from the inflow tube, and each slide was removed under a laminar flow hood and placed in a sterile 50-mL tube. After another saline rinse (2 mL), the surface of the slide was scraped repeatedly, and the scrapings and saline were collected in the tube. The tube was vortexed for 10 seconds, sonicated for 2 minutes, and vortexed again for 10 seconds to disperse the bacteria into suspension. The suspensions were then serially diluted and 100-µL aliquots applied to three plates containing TSA and incubated at 37°C for 24 hours. Colony-forming units (CFUs) were counted manually, and the number of CFUs per square centimeter was calculated. The resulting plate counts were log (10) transformed and expressed as the mean (± SD) value derived from plate counts from two DFR runs of three slides each.

For hydrodynamic treatment, the slides were removed from the DFR and placed in a glove box. The slides were placed in a holder and sprayed for approximately 20 seconds with about 150 mL of either saline or CAZS using a device that provided pressurized jet lavage. The spraying was done with both a side-to-side and an up-and-down sweeping motion so that all areas were sprayed twice, once in each axis. The slides were then placed in sterile 50-mL tubes, rinsed, scraped, dispersed, incubated and evaluated as described above.

The mean (± SD) percent reduction from control values in the quantity of *S. aureus* and *P. aeruginosa* bacteria (viz., the number of CFUs on each plate) after each treatment was calculated and the results assessed using two-sample *t* tests (MINITAB™ version 14, Minitab, State College, PA). A *P* value less than 0.05 was considered to represent a significant difference from the control value. The results are shown below in Table 1, expressed as the mean (± SD) number of colony-forming units per centimeter (log) derived from three plates assessed twice:

**Table 1**

| **Bacterial Plate Log Counts According to Type of Treatment** | | |
|---|---|---|
| **Treatment** | ***Staphylococcus aureus*** | ***Pseudomonas aeruginosa*** |
| None (Control) | | |
| | 8.7 ± 0.4 | 9.2 ± 0.2 |
| Static CAZS delivery | 6.2 ± 0.3 | 6.3 ± 1.3 |
| Hydrodynamic saline delivery | 6.4 ± 0.2 | 6.9 ± 0.1 |
| Hydrodynamic CAZS delivery | 4.8 ± 0.3 | 4.0 ± 0.5 |

The results in Table 1 show that significant bacterial biofilm removal was obtained. Before treatment, ample biofilms formed in the DFR cultures of both *S. aureus* and *P. aeruginosa*, with CFU counts for these Controls ranging from 7.8 to 9.5 log/cm². Static administration of CAZS resulted in a 2.5 log reduction (5.11 x 10⁸ to 1.65 x 10⁶; *P* = 0.001) in the number of *S. aureus* CFUs and a 2.9 log reduction (1.69 x 10⁹ to 1.91 x 10⁶; *P* = 0.002) in the number of *P. aeruginosa* CFUs. Mechanical disruption using hydrodynamic saline delivery alone decreased the number of *S. aureus* CFUs by 2.3 log units (5.11 x 10⁸ to 2.38 x 10⁶; *P* = 0.001) and the number of *P aeruginosa* CFUs by 2.4 log units (1.69 x 10⁹ to 7.31 x 10⁶; *P* = 0.001). However, mechanical disruption using hydrodynamic CAZS decreased the *S. aureus* CFU count by 3.9 log units (5.11 x 10⁸ to 6.37 x 10⁴; *P* = 0.001) and the *P. aeruginosa* CFU count by 5.2 log units (1.69 x 10⁹ to 1.04 x 10⁴; *P* = 0.001).

Confocal scanning laser microscopy (CSLM) was performed on three slides (for each treatment and bacteria species) not subjected to plate counts to allow imaging of the biofilm architecture in control and treated samples. The slides were stained for CSLM using a BACLIGHT™ Live/Dead kit (Molecular Probes, Invitrogen, Carlsbad, CA) containing two nucleic acid stains (SYTO 9, which detects living cells by fluorescing green, and propidium iodide, which detects dead cells by fluorescing red). After staining, the slides were examined using CSLM at a 630X magnification using a LEICA™ SP2 acoustic-optical beam splitter with a 2-photon MAI TAI™ attachment (Leica Microsystems, Bannockburn, IL) and fluorescence excitation and detection in both the green and red spectra. Each slide area was divided into 10 equally sized segments. A microscopic field was selected at random from each segment, and images were obtained at 1-µm intervals from the top of the biofilm to the substrate, thereby creating an image stack for each location. The CSLM analysis revealed that a thick biofilm carpeted the Control slides. Hydrodynamic treatment with saline and static treatment with CAZS decreased the amount of biofilm coverage markedly and reduced the organization of the remaining biofilm. Hydrodynamic treatment with CAZS produced a greater reduction both in biofilm coverage and in the amount of order in the biofilm community. The results corresponded generally to the plate count assessments with respect to the relative reductions in the amount of biofilm achieved with each treatment.

Of the three treatments investigated, power irrigation using CAZS and a pressurized jet lavage was the most effective in disrupting the bacterial biofilms. Power irrigation using saline had appreciable biofilm-reducing effects. However, the presence of a surfactant and citric acid in the irrigation solution significantly enhanced the reduction in CFU count in both *S. aureus* and *P. aeruginosa* biofilms. Large, statistically significant reductions occurred, with the mean decreases in bacterial plate counts being 3.9 and 5.2 log (a reduction of 10,000 to 100,000 times), respectively, for *S. aureus* and *P. aeruginosa* biofilms. A decrease of this magnitude *in vitro* indicates that an appropriate *in vivo* treatment in the middle or inner ear, nasal or sinus cavities or oral or pharyngeal tissues should effectively disrupt bacterial biofilms found there. Any remaining low level of persistent bacterial infection might be dealt with by host defenses or a topically or orally administered antimicrobial agent, and by application of a sealant as described above.

### Example 2 (comparative)

Experimental work conducted using *S aureus* and *P aeruginosa* cultures grown on TSA solid medium (viz., cultures made without use of HA-coated glass slides and the DFR and less likely to include a durable biofilm) indicates that a solvating system containing the surfactant but no metal ion sequestering agent may be less effective as a biofilm disruptor than a solvating system which also contains the metal ion sequestering agent. However, either solvating system may be a more effective biofilm disruptor than saline solution.

### Example 3

The CAZS solvating system employed in Example 1 was modified by replacing some of the water with gallium nitrate so that the modified system contained 25% gallium nitrate. A Control solution containing 25% gallium nitrate in deionized water was also prepared. When evaluated using the static treatment technique of Example 1, administration of the gallium nitrate Control solution resulted in a 3.4 log reduction (average of 4 runs) in the number of *S. aureus* CFUs and a 4.1 log reduction (average of 3 runs) in the number of *P. aeruginosa* CFUs. Static administration of the solution containing CAZS and gallium nitrate resulted in a 5.2 log reduction (average of 2 runs) in the number of *S. aureus* CFUs and a 5.5 log reduction (average of 2 runs) in the number of *P. aeruginosa* CFUs.

Although specific embodiments have been illustrated and described herein for purposes of description of the preferred embodiments, it will be appreciated by those of ordinary skill in the art that a wide variety of alternate or equivalent implementations calculated to achieve the same purposes may be substituted for the specific embodiments shown and described without departing from the scope of the present invention. This application is intended to cover any adaptations or variations of the preferred embodiments discussed herein. Therefore, it is manifestly intended that this invention be limited only by the claims.

## Claims

1. A medicament system which comprises:
(a) a solvating system comprising a metal ion sequestering agent and a surfactant that can detach, remove or otherwise disrupt at least a part of a biofilm attached or adhered to at least a portion of the middle or inner ear, to a surface within a nasal or sinus cavity, or to oral or pharyngeal tissue, and
(b) a polymeric film-forming medical sealant that can provide a protective layer over a site on which such a biofilm has been disrupted,
for use in a method for treating bacterial ear, nose or throat conditions, which method comprises:
(i) applying said solvating system to a treatment site comprising a bacterial biofilm attached or adhered to at least a portion of the middle or inner ear, to a surface within a nasal or sinus cavity, or to oral or pharyngeal tissue,
(ii) detaching, removing or otherwise disrupting at least a part of the biofilm, and then
(iii) applying said polymeric film-forming medical sealant to the treatment site to provide a protective layer that adheres to natural tissues at the site and resists detachment or other disruption until natural degradation or resorption of the sealant takes place.

2. A medicament system according to claim 1 for use in a method for treating bacterial ear, nose or throat conditions, wherein said treatment site is in a nasal or sinus cavity.

3. A medicament system according to claim 1 or claim 2 for use in a method for treating bacterial ear, nose or throat conditions, said method comprising applying the solvating system by spraying, lavage, misting, mopping, wicking or dripping; removing the thus-applied solvating system by flushing, rinsing, draining or absorption; and then applying the sealant by spraying, lavage, misting, mopping, wicking or dripping.

4. A medicament system for use according to claim 1 or claim 2 in a method for treating bacterial ear, nose or throat conditions, said method comprising applying the sealant as a film or conformal coating.

5. A medicament system for use according to any one of claims 1-4 wherein the surfactant comprises a zwitterionic surfactant.

6. A medicament system for use according to any one of claims 1-4 wherein the surfactant comprises an alkyl sulfate, alkyl sulfonate or aryl sulfonate.

7. A medicament system for use according to any one of claims 1-4 wherein the surfactant comprises a cationic surfactant.

8. A medicament system for use according to any one of claims 1-7 wherein the surfactant is at least 0.2% of the solvating system.

9. A medicament system for use according to any one of claims 1-7 wherein the surfactant is 0.3% to 30% of the solvating system.

10. A medicament system for use according to any one of claims 1-9 wherein the metal ion sequestering agent comprises a sequestering agent for sodium, calcium or iron.

11. A medicament system for use according to any one of claims 1-10 wherein the metal ion sequestering agent is present at a concentration of 0.01 to 0.5 M.

12. A medicament system for use according to any one of claims 1-11 wherein the solvating system further comprises water and a buffer and has a pH of 5 to 8.5.

13. A medicament system for use according to any one of claims 1-12 wherein the sealant hardens after application.

14. A medicament system for use according to any one of claims 1-13 wherein the sealant comprises a polysaccharide.

15. A medicament system for use according to claim 14 wherein the sealant comprises chitin, chitosan, starch, or a derivative thereof.

16. A medicament system for use according to any one of claims 1-15 wherein the solvating system or sealant further comprises an antimicrobial agent, or wherein the medicament system further comprises a separate antimicrobial agent.

17. A medicament system for use according to claim 1 wherein the solvating system and sealant will cause greater than a 1-log order reduction in an *in vitro* population of one or more of *Staphylococcus aureus, Pseudomonas aeruginosa, Streptococcus pneumonia, Haemophilus influenzae* or *Moraxella catarrhalis* bacteria.

18. A medicament system for use according to any one of claims 1-17 wherein the sealant further comprises an anti-fungal agent, antihistamine, steroidal or non-steroidal anti-inflammatory agent, anti-parasitic agent, antiviral agent, chemotherapeutic/antineoplastic agent, decongestant or mucolytic.

## Patentansprüche

1. Arzneimittelsystem, das Folgendes enthält:
(a) ein Solvatationssystem, das einen Metallionenkomplexbildner und eine oberflächenaktive Substanz, die zumindest einen Teil eines Biofilms ablösen, entfernen oder auf andere Weise unterbrechen kann, der zumindest an einem Abschnitt des Mittel- oder Innenohrs, an einer Fläche in einer Nasenhöhle oder in einer Nebenhöhle oder an einem Mund- oder Rachengewebe befestigt oder anhaftend ist, enthält, und
(b) einen medizinischen Dichtstoff, der einen Polymerfilm bildet, der über einem Ort, auf dem ein derartiger Biofilm unterbrochen wurde, eine Schutzschicht bereitstellen kann,
zur Verwendung in einem Verfahren zum Behandeln bakterieller Ohr-, Nasen- oder Rachenbedingungen, wobei das Verfahren Folgendes umfasst:
(i) Aufbringen des Solvatationssystems auf einen Behandlungsort, der einen bakteriellen Biofilm umfasst, der zumindest an einem Abschnitt des Mittel- oder Innenohrs, an einer Fläche in einer Nasenhöhle oder in einer Nebenhöhle oder an einem Mund-oder Rachengewebe befestigt oder anhaftend ist,
(ii) Ablösen, Entfernen oder auf andere Weise Unterbrechen zumindest eines Teils des Biofilms, und darauffolgend
(iii) Aufbringen des medizinischen Dichtstoffs, der einen Polymerfilm bildet, auf den Behandlungsort, um eine Schutzschicht bereitzustellen, die an den natürlichen Geweben am Ort haftet und einem Ablösen oder einem anderweitigen Unterbrechen widersteht, bis die natürliche Zersetzung oder Resorption des Dichtstoffs stattfindet.

2. Arzneimittelsystem nach Anspruch 1 zur Verwendung in einem Verfahren zum Behandeln von bakteriellen Bedingungen in Ohr, Nase oder Rachen, wobei der Behandlungsort in einer Nasenhöhle oder in einer Nebenhöhle ist.

3. Arzneimittelsystem nach Anspruch 1 oder Anspruch 2 zur Verwendung in einem Verfahren zum Behandeln von bakteriellen Bedingungen in Ohr, Nase oder Rachen, wobei das Verfahren das Aufbringen des Solvatationssystems durch Sprühen, durch eine Spülung, durch Vernebeln, durch Wischen, durch Dochtwirkung oder durch Tropfen; das Entfernen des somit aufgebrachten Solvatationssystems durch Spülen, Waschen, Abtropfenlassen oder Absorption; und darauffolgend das Aufbringen des Dichtstoffs durch Sprühen, durch eine Spülung, durch Vernebeln, durch Wischen, durch Dochtwirkung oder durch Tropfen umfasst.

4. Arzneimittelsystem zur Verwendung nach Anspruch 1 oder Anspruch 2 in einem Verfahren zum Behandeln von bakteriellen Bedingungen in Ohr, Nase oder Rachen, wobei das Verfahren das Aufbringen des Dichtstoffs als einen Film oder als einen gleichmäßigen Überzug umfasst.

5. Arzneimittelsystem zur Verwendung nach einem der Ansprüche 1-4, wobei die oberflächenaktive Substanz eine zwitterionische, oberflächenaktive Substanz enthält.

6. Arzneimittelsystem zur Verwendung nach einem der Ansprüche 1-4, wobei die oberflächenaktive Substanz ein Alkylsulfat, ein Alkylsulfonat oder ein Arylsulfonat enthält.

7. Arzneimittelsystem zur Verwendung nach einem der Ansprüche 1-4, wobei die oberflächenaktive Substanz eine kationische, oberflächenaktive Substanz enthält.

8. Arzneimittelsystem zur Verwendung nach einem der Ansprüche 1-7, wobei die oberflächenaktive Substanz zumindest 0,2 % des Solvatationssystems bildet.

9. Arzneimittelsystem zur Verwendung nach einem der Ansprüche 1-7, wobei die oberflächenaktive Substanz 0,3 % bis 30 % des Solvatationssystems bildet.

10. Arzneimittelsystem zur Verwendung nach einem der Ansprüche 1-9, wobei der Metallionenkomplexbildner einen Komplexbildner für Natrium, Kalzium oder Eisen enthält.

11. Arzneimittelsystem zur Verwendung nach einem der Ansprüche 1-10, wobei der Metallionenkomplexbildner mit einer Konzentration von 0,01 bis 0,5 M vorhanden ist.

12. Arzneimittelsystem zur Verwendung nach einem der Ansprüche 1-11, wobei das Solvatationssystem ferner Wasser und einen Puffer enthält und einen pH-Wert von 5 bis 8,5 aufweist.

13. Arzneimittelsystem zur Verwendung nach einem der Ansprüche 1-12, wobei der Dichtstoff nach dem Aufbringen aushärtet.

14. Arzneimittelsystem zur Verwendung nach einem der Ansprüche 1-13, wobei der Dichtstoff ein Polysaccharid enthält.

15. Arzneimittelsystem zur Verwendung nach Anspruch 14, wobei der Dichtstoff Chitin, Chitosan, Stärke oder ein Derivat davon enthält.

16. Arzneimittelsystem zur Verwendung nach einem der Ansprüche 1-15, wobei das Solvatationssystem oder der Dichtstoff ferner ein antimikrobielles Mittel enthalten oder wobei das Arzneimittelsystem ferner ein separates, antimikrobielles Mittel enthält.

17. Arzneimittelsystem zur Verwendung nach Anspruch 1, wobei das Solvatationssystem und der Dichtstoff eine Verringerung von mehr als einer 1. logarithmischen Ordnung in einer *In*-*Vitro*-Population von *Staphylococcus aureus* Bakterien und/oder *Pseudomonas aeruginosa* Bakterien und/oder *Streptococcus pneumonia* Bakterien und/oder *Haemophilus influenzae* Bakterien und/oder *Moraxella catarrhalis* Bakterien bewirken.

18. Arzneimittelsystem zur Verwendung nach einem der Ansprüche 1-17, wobei das Dichtmittel ferner ein Antipilzmittel, ein Antihistaminikum, ein steroides oder nicht steroides entzündungshemmendes Mittel, ein antiparasitäres Mittel, ein Antivirenmittel, ein chemotherapeutisches/antineoplastisches Mittel, ein abschwellendes Mittel oder ein schleimlösendes Mittel enthält.

## Revendications

1. Système médicamenteux qui comporte :
(a) un système de solvatation comportant un agent chélateur d'ions métalliques et un tensio-actif qui peut détacher, retirer ou sinon interrompre au moins une partie d'un biofilm fixé ou adhérant à au moins une portion de l'oreille moyenne ou interne, à une surface à l'intérieur d'une cavité nasale ou sinusale, ou à un tissu oral ou pharyngien, et
(b) un agent d'étanchéité médical formant un film polymère qui peut fournir une couche protectrice au-dessus d'un site sur lequel un tel biofilm a été interrompu,
pour une utilisation dans un procédé destiné à traiter des conditions bactériennes de l'oreille, du nez ou de la gorge, lequel procédé comporte les étapes consistant à :
(i) appliquer ledit système de solvatation à un site de traitement comportant un biofilm bactérien fixé ou adhérant à au moins une portion de l'oreille moyenne ou interne, à une surface à l'intérieur d'une cavité nasale ou sinusale, ou à un tissu oral ou pharyngien,
(ii) détacher, retirer ou sinon interrompre au moins une partie du biofilm, et ensuite
(iii) appliquer ledit agent d'étanchéité médical formant un film polymère au site de traitement pour fournir une couche protectrice qui adhère à des tissus naturels sur le site et résiste à un détachement ou une autre interruption jusqu'à ce qu'une dégradation ou une résorption naturelle de l'agent d'étanchéité ait lieu.

2. Système médicamenteux selon la revendication 1 pour une utilisation dans un procédé destiné à traiter des conditions bactériennes de l'oreille, du nez ou de la gorge, dans lequel ledit site de traitement se situe dans une cavité nasale ou sinusale.

3. Système médicamenteux selon la revendication 1 ou la revendication 2 pour une utilisation dans un procédé destiné à traiter des conditions bactériennes de l'oreille, du nez ou de la gorge, ledit procédé comportant l'application du système de solvatation par pulvérisation, lavage, brumisation, essuyage, effet de capillarité ou égouttement ; le retrait du système de solvatation ainsi appliqué par nettoyage à grande eau, rinçage, drainage ou absorption ; et ensuite l'application de l'agent d'étanchéité par pulvérisation, lavage, brumisation, essuyage, effet de capillarité ou égouttement.

4. Système médicamenteux pour une utilisation selon la revendication 1 ou la revendication 2 dans un procédé destiné à traiter des conditions bactériennes de l'oreille, du nez ou de la gorge, ledit procédé comportant l'application de l'agent d'étanchéité sous la forme d'un film ou d'un revêtement enrobant.

5. Système médicamenteux pour une utilisation selon l'une quelconque des revendications 1 à 4, dans lequel le tensio-actif comporte un tensio-actif zwitterionique.

6. Système médicamenteux pour une utilisation selon l'une quelconque des revendications 1 à 4, dans lequel le tensio-actif comporte un sulfate d'alkyle, un sulfonate d'alkyle ou un sulfonate d'aryle.

7. Système médicamenteux pour une utilisation selon l'une quelconque des revendications 1 à 4, dans lequel le tensio-actif comporte un tensio-actif cationique.

8. Système médicamenteux pour une utilisation selon l'une quelconque des revendications 1 à 7, dans lequel le tensio-actif représente au moins 0,2 % du système de solvatation.

9. Système médicamenteux pour une utilisation selon l'une quelconque des revendications 1 à 7, dans lequel le tensio-actif représente 0,3 % à 30 % du système de solvatation.

10. Système médicamenteux pour une utilisation selon l'une quelconque des revendications 1 à 9, dans lequel l'agent chélateur d'ions métalliques comporte un agent chélateur pour le sodium, le calcium ou le fer.

11. Système médicamenteux pour une utilisation selon l'une quelconque des revendications 1 à 10, dans lequel l'agent chélateur d'ions métalliques est présent à une concentration de 0,01 à 0,5 M.

12. Système médicamenteux pour une utilisation selon l'une quelconque des revendications 1 à 11, dans lequel le système de solvatation comporte en outre de l'eau et un tampon et a un pH de 5 à 8,5.

13. Système médicamenteux pour une utilisation selon l'une quelconque des revendications 1 à 12, dans lequel l'agent d'étanchéité durcit après application.

14. Système médicamenteux pour une utilisation selon l'une quelconque des revendications 1 à 13, dans lequel l'agent d'étanchéité comporte un polysaccharide.

15. Système médicamenteux pour une utilisation selon la revendication 14, dans lequel l'agent d'étanchéité comporte de la chitine, du chitosane, de l'amidon et un dérivé de ceux-ci.

16. Système médicamenteux pour une utilisation selon l'une quelconque des revendications 1 à 15, dans lequel le système de solvatation ou l'agent d'étanchéité comporte en outre un agent antimicrobien, ou dans lequel le système médicamenteux comporte en outre un agent antimicrobien séparé.

17. Système médicamenteux pour une utilisation selon la revendication 1, dans lequel le système de solvatation et l'agent d'étanchéité entraîneront une réduction supérieure à une réduction d'ordre 1-log dans une population *in vitro* d'une ou plusieurs bactéries parmi les bactéries *Staphylococcus aureus, Pseudomonas aeruginosa, Streptococcus pneumonia, Haemophilus influenzae* ou *Moraxella catarrhalis.*

18. Système médicamenteux pour une utilisation selon l'une quelconque des revendications 1 à 17, dans lequel l'agent d'étanchéité comporte en outre un agent antifongique, un antihistaminique, un agent anti-inflammatoire stéroïdien ou non stéroïdien, un agent antiparasitique, un agent antiviral, un agent chimiothérapeutique/antinéoplastique, un décongestionnant ou un mucolytique.
